(19) Europäisches Patentamt

European Patent Office

Office européen des brevets

(11) **EP 0 975 342 B1**

(12) **EUROPEAN PATENT SPECIFICATION**

(45) Date of publication and mention
of the grant of the patent:
**25.06.2003  Bulletin 2003/26**

(51) Int Cl.⁷: **A61K 31/43**, A61K 9/50,
A61K 9/20

(21) Application number: **98912303.9**

(22) Date of filing: **10.02.1998**

(86) International application number:
**PCT/EP98/00811**

(87) International publication number:
**WO 98/035672 (20.08.1998 Gazette 1998/33)**

(54) **PHARMACEUTICAL FORMULATIONS COMPRISING AMOXOCYLLIN AND CLAVULANATE**

AMOXICILLIN UND CLAVULANAT ENTHALTENDE ARZNEIZUBEREITUNGEN

FORMULATIONS PHARMACEUTIQUES COMPRENANT DE L'AMOXYCILLINE ET DU
CLAVULANATE

(84) Designated Contracting States:
**AT BE CH DE DK ES FI FR GB GR IE IT LI LU MC
NL PT SE**
Designated Extension States:
**RO SI**

(30) Priority: **14.02.1997  GB 9703099
14.02.1997  GB 9703100
14.02.1997  GB 9703101**

(43) Date of publication of application:
**02.02.2000  Bulletin 2000/05**

(60) Divisional application:
**01201884.2 / 1 142 574**

(73) Proprietors:
- **Laboratoire GlaxoSmithKline S.A.S.
78163 Marly-le-Roi Cedex (FR)**
- **Smithkline Beecham S.A.
Madrid (ES)**
- **SMITHKLINE BEECHAM CORPORATION
Philadelphia Pennsylvania 19103 (US)**

(72) Inventors:
- **MENTION, Jacky Andre Gustave,
SmithKline Beecham
F-53101 Mayenne Cedex (FR)**
- **SANROMA BORDALLO Jose Luis,
SmithKline Beecham SA.
E-45007 Toledo (ES)**
- **STORM, Kevin,
SmithKline Beecham Pharmaceuticals
Bristol, TN 37620 (US)**

(74) Representative:
**Connell, Anthony Christopher et al
GlaxoSmithKline
Corporate Intellectual Property (CN9.25.19)
980 Great West Road
Brentford, Middlesex TW8 9GS (GB)**

(56) References cited:
**WO-A-92/19227          WO-A-94/27557
WO-A-95/20946          WO-A-95/25516
WO-A-95/28148          WO-A-95/28927
GB-A- 2 005 538**

**Description**

The present invention relates to novel formulations comprising amoxycillin and clavulanate (co-amoxiclav), to processes for their preparation and their use in therapy.

The product co-amoxiclav is marketed by SmithKline Beecham as Augmentin for treating bacterial infections. It comprises a combination of the β-lactam antibacterial agent amoxycillin (present as the trihydrate) and the β lactamase inhibitor clavulanic acid (present as the potassium salt). Various formulations, for instance tablets, capsules, powders (for reconstitution into aqueous syrups) and sachets containing free flowing granules, are provided, containing different ratios of amoxycillin and clavulanic acid. Thus, tablets and powders (for reconstitution into aqueous syrups) are available comprising amoxycillin and clavulanic acid in ratios of 2:1, 4:1 and 7:1, whilst sachets are available with the same ratios, as well as 8:1 (Augmentin, SmithKline Beecham, France, poudre pour suspension buvable a 100mg/ml nourisson', comprising 100mg amoxycillin and 12.5mg clavulanate). This leads to a complexity in the manufacturing process as each product with a different ratio is prepared separately, by blending together the different amounts of amoxycillin and clavulanate at an early stage of the process.

In addition, clavulanate has been blended with a diluent, conventionally in a 1:1 ratio, for safer storage and transportation. This has been silica gel (for instance the product Syloid AL-1) for non-tablet formulations and microcrystalline cellulose (for instance the product Avicel) for tablet formulations, adding to further complexity in the manufacturing process.

There therefore remains the need to devise more efficient manufacturing processes, to reduce complexity and improve the economics of the process. It has now been found that this can be achieved by the use of a common intermediate granulate comprising a fixed ratio of amoxycillin and clavulanate, with the differing ratios in the final formulation then being achieved by adding in appropriate amounts of amoxycillin at a later stage in the process. Formulations having granulates comprising amoxycillin and clavulanate are described in GB 2 005 538-A (Beecham Group), WO 95/28927 (SmithKline Beecham) and WO 95/25516 (SmithKline Beecham). There is however no suggestion of combining granulates of amoxycillin and clavulanate with granulates of amoxycillin.

As the absolute amount of drug substance increases, the tablet size also increases, making the tablets less attractive to swallow. Tablets further comprise excipients such as disintegrants, diluents, lubricants which are necessary to allow the tablet to be manufactured and to enhance the pharmacokinetic performance of the tablets, once taken. Such excipients further add to the gross weight of a tablet. Furthermore, the conventional practice of formulating tablets comprising potassium clavulanate from a 1:1 blend of potassium clavulanate and a diluent, referred to above, further adds to the weight and size of a tablet. Thus, currently available (coated) tablets comprising 500/125 and 875/125 mg amoxycillin plus clavulanate (expressed as the weight of the corresponding free acids) have weights of 1050 and 1482mg, respectively, of which the drug substances account for about 70% and 81% respectively, by weight. Prototype formulations disclosed in earlier patent applications suggest 500/125mg tablets weighing 872mg (uncoated) (Example 1, GB 2 005 538-A), 950mg (uncoated), 1050mg (coated) (Example 14 &15, WO 92/19227) and 875/125mg tablets weighing 1350mg (uncoated) and 1450mg (coated) (Example 14 &15, WO 92/19227). There is therefore a need to provide tablet formulations having a smaller size for a given amount of drug substance.

A new co-amoxiclav dosage regimen of 1000/125 mg twice a day (bd) has generated a need for an appropriate tablet formulation(s). This unit dose has not so far been provided as a tablet formulation as merely increasing the size of the exisiting 875/125mg tablet was considered to give a tablet which would be unacceptably large for the patient to swallow..

Accordingly, in a first aspect, the present invention provides a granulate comprising amoxycillin trihydrate and potassium clavulanate in a ratio of 1:1, 2:1 or 4:1, wherein this ratio refers to the weight of parent compound amoxycillin or clavulanic acid, and an intragranular diluent present in from 5 to 25% by weight of potassium clavulanate.

Suitable intragranular diluents are silica gel (which may also act as an intragranular dessicant), for instance the product Syloid AL-1, or microcrystalline cellulose (which may also act as a compression aid), for instance the product Avicel. Preferably, the intragranular diluent, if present, is silica gel on account of its superior dessicating power. Conventionally, a diluent is included in a weight ratio of about 1:1 with respect to the weight of potassium clavulanate, the granulate being prepared from a 1:1 blend of potassium clavulanate and silica gel or microcrystalline cellulose. It has however also been found possible to reduce the relative to amount of intragranular diluent, in particular silica gel, to a much lower level, and even avoid the use thereof. Accordingly, in a further aspect the invention provides for a granulate comprising amoxycillin and clavulanate as hereinbefore defined and an intragranular disintegrant present in from 5 to 15%, typically about 10% by weight of potassium clavulante. Preferably, the dessicant diluent is included in a proportion of about 0.5-5% by weight of the amoxycillin plus clavulanate, more preferably from 1 to 3%. Such granulates allow the preparation of final formulations, in particular tablets, having a reduced overall weight and size.

In a further aspect, the present invention provides a pharmaceutical formulation comprising amoxycillin and clavulanate in a ratio of n:1 where n is a number from 1 to 16 which comprises:

a first set of granulates comprising amoxycillin and clavulanate in a ratio of 1:1, 2:1 or 4:1; and

a second set of granulates comprising amoxycillin and no clavulanate;

in a ratio between the first and second sets of granulates to give an overall ratio between amoxycillin and clavulanate of n:1.

Preferably, n is 2, 4, 6, 7, 8, or 14.

Preferably, the first set of granulates comprise amoxycillin and clavulanate in a ratio of 2:1.

In a further aspect, the present invention provides for a range of pharmaceutical formulations comprising different ratios of amoxycillin and clavulanate from 2:1 to 14:1, for instance selected from 2:1, 4:1, 6:1, 7:1, 8:1 and 14:1, which is prepared from a first set of granulates having a fixed ratio of amoxycillin and clavulanate, for instance 1:1 or 2:1, preferably 2:1, and a second set of granulates comprising amoxycillin, by combining different relative proportions of the two sets of granulates.

Thus, for instance, a range comprising formulations having ratios of 4:1, 7:1 and 8:1 may be prepared by combining granulates comprising amoxycillin and clavulanate in the ratio 2:1 with different relative amounts of granulates comprising amoxycillin, for instance in the ratios 3:2, 3:5 and 3:6 respectively.

The terms "amoxycillin" and "clavulanate" used herein, and unless otherwise specified, include both the free parent acids and derivatives such as salts thereof.

Weights and ratios are expressed in terms of the weight of parent compound amoxycillin or clavulanic acid, this terminology being used throughout this description unless otherwise stated.

Suitable derivatives of amoxycillin are amoxycillin trihydrate, anhydrous amoxycillin and alkali metal salts of amoxycillin such as sodium amoxycillin. Preferably, the equilibrium relative humidity (ERH) of the amoxycillin trihydrate used as a raw material for granulate production is carefully controlled by appropriate drying so that it doe not compromise other aspects of the formulation. Preferably, the ERH is less than 50%, more preferably less than 30%, most preferably from 10 to 20%.

Suitable derivatives of clavulanic acid are alkali metal salts of clavulanic acid such as potassium clavulanate.

Preferably, formulations of this invention comprise amoxycillin trihydrate and potassium clavulanate, this combination having met with regulatory approval, and being particularly advantageous.

Suitable granulates for use in formulations of the present invention have been previously described in WO 92/19277 (SmithKline Beecham) and GB 2 005 538-A (Beecham Group). Suitable granulates may comprise in addition to amoxycillin and, if present, clavulanate, excipients conventionally used in such granulates, for instance an intragranular disintegrant, intragranular lubricant and intragranular diluent.

Suitable intra-granular disintegrants include starches, such as maize starch and rice starch, crospovidone (cross-linked N-vinyl-2-pyrrolidinone; CLPVP), sodium starch glycollate, croscarmellose sodium and formaldehyde - casein, or combinations thereof. Preferred intra-granular disintegrants include sodium starch glycollate and CLPVP, in particular CLPVP, for example the product marketed under the trade names Polyplasdone XL and Polyplasdone XL-10. Preferably, the intragranular disintegrant is present in from 0.1 to 10%, preferably from 1.0 to 8.0%, more preferably from 1.25 to 3.5% by weight of the granulate.

Suitable intragranular lubricants are those conventional to the art, such as long-chain fatty acids, such as stearic acid, or salts thereof, in particular Group II metal salts, such as of magnesium or calcium. A preferred lubricant is magnesium stearate. Preferably, a lubricant is used in as low a proportion for instance form 0 tol%, preferably from 0 to 0.5%, more preferably from 0 to 0.35%. Preferably, if the granulate is formed from ingredients which have been compacted using a roller compactor, it is possible to use no lubricant at all.

Typically the proportion of amoxycillin and potassium clavulanate, if present, in a granulate is from 90 to 99.9wt %, preferably from 92 to 99wt %, for instance from 95 to 99wt %, such as 96.5 to 98.75% of the weight of the granulate. When the granulate contains a diluent, this may comprise up to 30wt % of the granulate, preferably, up to 25wt%, more preferably up to 10wt%, preferably up to 5wt%. When the granulate contains a diluent, the granulate will contain a correspondingly lower proportion amoxycillin or amoxycillin plus clavulanate, for example from 70 to 99.9wt % of the granulate, preferably from 85%, more preferably from 90%, typically from 93 to 97wt%.

Preferred amoxycillin granulates comprise from 95 to 99wt%, preferably from 96 to 98wt%, more preferably from 96.5 to 97.5wt% amoxycillin trihydrate and from 1 to 5wt%, preferably 2 to 4wt%, more preferably from 2.5 to 3.5wt% of starch glycollate or CLPVP. Most preferred granulates consist essentially of about 97wt% amoxycillin trihydrate and 3wt% CLPVP.

Preferred amoxycillin and potassium clavulanate granulates comprise a 2:1 ratio of amoxycillin to clavulanate; silica gel present in from 5 to 15wt% of potassium clavulanate, typically about 10wt%; and CLPVP present in from 0.5 to 5wt% of amoxycillin trihydrate, preferably 1 to 4wt%, typically about 3%.

Preferably the particles of amoxycillin and clavulanate, if present, in the granulates are in the size range 1µm to 300µm, especially 10µm to 200µm. A typical suitable size distribution of the amoxycillin/clavulanic acid particles is : >200µ, 5% or less; 200-100µ, 5-15%; 100-50µ, 7.5-15%; <50µ, 70% or more.

Granulates according to the present invention may be presented in a variety of finished formulations, including, for instance, tablets, for example, swallow tablets, dispersible tablets and chewable, optionally effervescent, tablets; in capsules; aqueous syrups and sachets. These may be prepared by combining the granulates with additional, extranular, excipients conventionally used in such formulations and further processing into finished formulations.

Tablets of the present invention may include an extra-granular disintegrant, for instance maize-starch and rice starch, CLPVP, sodium starch glycollate, croscarmellose sodium, microcrystalline or microfine cellulose, low-substituted hydroxypropylcellulose (i.e. cellulose partially substituted with 2-hydroxypropyl groups, e.g. less than 25% substituted, preferably 7-16% substituted), cross-linked sodium carboxymethylcellulose, swellable ion exchange resins, formaldehyde-casein, or alginates. A preferred extra-granular disintegrant is low-substituted hydroxypropylcellulose, for instance the product L-HPC LH-11 (from Shinetsu). This also has useful binding properties, allowing the preparation of tablets with good hardness for a relatively lower proportion of ingredient. Other useful extra-granular disintegrants include CLPVP and sodium starch glycollate.

The proportion of extra-granular disintegrant to total tablet weight may vary between broad limits, for example 0.1-25 weight %. For example, low-substituted hydroxypropylcellulose, CLPVP or sodium starch glycollate may preferably be used in a proportion 0.1-15.0 weight %, preferably 1.0 10.0 weight %, preferably 3.0-8.0 weight % of the total tablet weight. If cellulose or a combination containing cellulose is used, e.g. as described above containing around 80-90% by weight of cellulose, the extra-granular disintegrant may comprise 1-20 weight %. Dispersible tablets will tend to comprise a relatively higher proportion of extra-granular disintegrant, to aid the dissolution process.

Tablets of the present invention may also include an extra-granular lubricant, for instance a long-chain fatty acid, such as stearic acid, or salts thereof, in particular Group II metal salt thereof, such as a magnesium or calcium salt, preferably magnesium stearate. Preferably, the extra-granular lubricant is used in from 0.1 to 2%, more preferably from 0.2 to 0.5, typically, 0.3 to 0.4 weight% of the tablet.

Formulations of the present invention may also include an extra-granular dessicant such as silica gel. This may be present in an amount up to 5% by weight of the formulation, preferably, up to 2% of a tablet formulation. Careful control of the Equilibrium Relative Humidity of the amoxycillin trihydrate used for the granulates allows the use of a relatively lesser proportion of an extra-granular dessicant.

Tablets may also include other conventional excipients, typically present up to about 10% of the total tablet weight, for instance flavoring agents, for example flavorings such as menthol, peppermint, vanilla or fruit flavorings, flavoring agents typically being present up to around 0.5-5% by weight of the whole tablet and sweeteners, e.g. aspartame, present of up to around 15mg per unit dose. Excipients may also include colouring agents, preservatives, suspending aids and fillers such as silicon dioxide, microcrystalline cellulose, dicalcium phosphate, lactose, sorbitol, calcium carbonate or magnesium carbonate. Such excipients are preferably mixed with the extra-granular disintegrant and lubricant (if present). The materials present in the tablets should have low free moisture content and preferably be pre-dried.

Tablets of the present invention may also contain an effervescent couple of known type, e.g. a solid acid and an alkali metal carbonate or bicarbonate which generates carbon dioxide on contact with water to assist in disintegration of the tablet, for instance monosodium citrate (56.04% w/w) and sodium bicarbonate(43.96% w/w). Preferably, the couple is provided as granules prepared from anhydrous powdered monosodium citrate and powdered sodium bicarbonate and compacted together by roller compaction, according to the process described in WO 97/02014 (SmithKline Beecham Laboratoires Pharmaceutiques).

The tablets may be film coated in a conventional manner, e.g. for cosmetic, palatability or production purposes. Suitable coatings include hydroxypropylcellulose, acrylate and/or methacrylate co-polymers such as the products available under the trade mark Eudragit, resins etc. Alternatively the coating may be an enteric coating, e.g. which is insoluble in acidic gastric juice but soluble in alkaline digestive juice. Such a coating enables the tablet to pass through the stomach into the duodenum, from where it is absorbed. Suitable enteric coatings include cellulose acetate phthalate.

Preferably, a film coating is applied by aqueous film coating techniques, thereby avoiding the need for organic solvents. Suitable polymers for use in such techniques include hydroxypropylcellulose, hydroxypropylmethyl cellulose, ethylcellulose (for example ethylcellulose in a latex composition as supplied by the FMC Corporation as "Aqua-Coat" (trade mark)), methylhydroxyethylcellulose, polyvinylpyrrolidone ("PVP", e.g. as supplied under the name Povidone (trade mark), sodium carboxymethylcellulose and acrylate polymers (e.g. the known methacrylic acid esters supplied under the trade name "Eudragit" (trade mark)).

A preferred polymer is hydroxypropylmethylcellulose ("HPMC") preferably in combination with a polyethylene glycol ("PEG"). PEG's of low molecular weight (200 to 600 series) are liquid at room temperature and find use as plasticisers. PEG's with high molecular weights (900 to 8000) are waxy solids at room temperature and are used in combination with low molecular weight PEG's and with other polymers such as HPMC to modify film properties and to contribute to tablet sheen.

A preferred polymer which can be applied by aqueous film coating techniques is one or more hydroxypropylmethyl celluloses combined with one or more PEG's. HPMC polymers have the advantages of solubility in physiological fluids as well as water, non-interference with tablet disintegration, dissolubility or drug availability, formation of a flexible film, freedom from objectionable taste or odour, stability to heat, light, air, moisture, compatibility to stabilisers, colourants opacifiers, and gloss. The hydroxypropylmethylcellulose functions as a film former, and the polyethylene glycol functions as a plasticiser. The hydroxypropylmethyl cellulose: polyethylene glycol ratio in the film coating is preferably between 7.5 : 1 to 5.5 : 1, e.g. around 6.5 : 1 ±10%.

Preferably the hydroxypropylmethyl cellulose is applied in the form of a mixture of hydroxypropylmethyl cellulose 6 cps and 15 cps, in a ratio of around 2:1 to 4:1 e.g. around 3:1 ±10%. Preferably the polyethylene glycol is applied in the form of a mixture of polyethylene glycol 4000 and 6000 in a ratio between around 1:2 to 2:1, e.g. around 1:1. The film coat may also preferably include an opacifier, for example titanium dioxide (white). Preferably the opacifier may be present in around a 1:1 ± 10% proportion with the hydroxypropylmethyl cellulose in the film coat.

The materials of the film coat are preferably applied by an aqueous film coating process, as application in this way form a film of a nature which also appears to contribute to the improved consistency in bioavailability. A suitable solids loading for the aqueous film coat is around 10-30% w/v, typically 10-20%, e.g. 15% ± 2%.

Preferably the film coating is applied so as to deposit a weight of dried film materials corresponding to between 0.5 and 5%, preferably 1 and 4%, more preferably 1.5 to 3.5% of the total coated tablet weight.

Preferably the proportion of amoxycillin and clavulanate in tablets of the present invention is 60-98% by weight of the total tablet (calculated as the weight of the particular form used, for instance the weight of amoxycillin trihydrate or potassium clavulanate).

Preferably, tablets according to the present invention are provided in convenient unit dosage forms, for instance comprising nominally 125/62.5, 250/62.5, 250/125, 500/62.5, 500/125, 875/125 and 1000/125mg amoxycillin/ clavulanate. The tablets may be dispersed in water prior to ingestion, or may alternatively be chewed or swallowed whole.

The skilled man will readily appreciate that, in tablets of this invention, the granulates may be in a crushed state resulting from the compaction of the tablet, and consequently may not have discrete boundaries, or may be subdivided or broken up into smaller granulates. The invention is intended to include tablets having such a structure containing crushed granulates. Preferably the size of the granulates is in the range 100μm to 2mm, preferably around 1mm ± 0.25mm, maximum dimension.

Preferably the tablets are packaged in a container that inhibits the ingress of atmospheric moisture, e.g. blister packs or tightly closeable bottles etc. as conventional in the art. Preferably bottles also include a desiccant material to preserve the clavulanate.

By reducing the relative amount of excipients used, in particular intra-granular diluent, as well as minimising the amounts of extra-granular excipients used in a tablet formulation, tablets of reduced weight may be prepared. This is assisted by careful control of the moisture content of the various excipients, as well as amoxycillin trihydrate, to ensure that a larger dessicating capacity is not required. Accordingly, in a further aspect, the present invention provides for a tablet formulation comprising a first set of granulates comprising amoxycillin and clavulanate with a second set of granulates comprising amoxycillin and no clavulanate as hereinbefore defined in which the combined weight of all excipients is less than 20%, preferably less than 18%, more preferably less than 15%, typically from 10 to 12%, of the uncoated core weight of the tablet. This provides tablets which are easier for the patient to swallow. In addition, such reduced weight tablets cost less to produce as less raw materials are used, less material has to be transported around a manufacturing plant and the capacity of the equipment is effectively increased, since less material is processed per tablet.

Preferred reduced weight tablets comprise:

amoxycillin granulates which consist essentially of amoxycillin trihydrate present in about 97wt% and CLPVP present in about 3wt%;
amoxycillin and potassium clavulanate granulates which comprise a 2:1 ratio of amoxycillin to clavulanate; silica gel present in about 10wt% of potassium clavulanate and about 3wt% of CLPVP; and
extra granular excipients including:

silica gel (preferably 0.5 to 2.0%, more preferably about 1.0 to 1.2%); low-substituted hydroxypropylcellulose (preferably 3 to 8%, more preferably 5 to 6%); colloidal silica (preferably 0.1 to 0.5%, more preferably 0.1 to 0.3%); and magnesium stearate (preferably 0.2 to 0.5%, more preferably 0.3 to 0.4%); or
CLPVP (preferably 3 to 10%, more preferably 5 to 8%); and magnesium stearate (preferably 0.2 to 0.5%, more preferably 0.3 to 0.4%).

Preferred 500/62.5mg tablets will have tablet core weights in the range 700 to 800mg, more preferably 720 to 780mg. Preferred 500/125mg tablets will have tablet core weights in the range 800 to 950mg, more preferably 800 to 900mg, most preferably 800 to 850mg. Preferred 875/125mg tablets will have tablet core weights in the range 1250 to 1375mg, more preferably 1250 to 1325mg. Preferred 1000/125mg tablets will have tablet core weights in the range 1400 to 1550mg, more preferably 1425 to 1475mg.

Especially preferred 500/125mg tablets will have a total weight (core plus coating) of less than 900mg, most preferably less than 850mg. Especially preferred 500/62.5mg tablets will have a total weight (core plus coating) of less than 850mg, most preferably less than 800mg. Especially preferred 875/125mg tablets will have a total weight (core plus coating) of less than 1400mg, most preferably less than 1350mg. Especially preferred 1000/125mg tablets will have a total weight (core plus coating) of less than 1550mg, preferably less than 1500mg.

The skilled person will readily appreciate that the above mentioned tablets may also be produced by a more conventional approach, using a single set of granules comprising the amoxycillin and clavulanate in the final ratio of, for instance, 4:1, 7:1 or 8:1, rather than the two sets of granules hereinbefore described, using intra- and extra-granular excipients in the same proportions. The present invention covers all such tablets.

As hereinbefore described, a 1000/125mg unit dosage in a tablet formulation may be provided as a single, preferably reduced weight, tablet. Preferred reduced weight 1000/125mg tablets comprise amoxycillin and clavulanate present in from 83 to 95%, preferably 85 to 93%, more preferably, 87 to 91% of the core tablet weight and pharmaceutically acceptable excipients present in from 5 to 17%, preferably 7 to 15%, more preferably, 9 to 13% by weight of the core weight of the tablet (excluding any coating). The skilled person will however readily appreciate that the dosage may also be provided using two 500/62.5mg tablets (ratio amox/clav = 8:1). Such tablets will be intrinsically smaller as they contain less drug substance so such tablets may also be based on more conventional formulations, for instance existing 500/125mg formulations where there is not such a need to reduce the weight and size of the tablet. Accordingly, in a further aspect, the present invention provides for a tablet comprising about 500mg amoxycillin and about 62.5mg potassium clavulanate and pharmaceutically acceptable excipients. The skilled person will further appreciate that a 1000/125mg dosage may also be provided by the combination of a 500/125mg amoxycillin/clavulanate tablet and a 500mg amoxycillin tablet, for instance using existing such tablets or adapting one or the other to more readily distinguish between the two, for instance by shape or colour. Patient compliance with such two tablet strategies may be enhanced by providing the tablets in a blister pack presentation, with each blister containing two tablets. The present invention covers all such approaches.

A 1000/125mg unit dosage may also be provided as a chewable, optionally effervescent tablet, whereby the problem of having to swallow whole a large tablet is overcome by the patient chewing the tablet and breaking it down into smaller pieces prior to swallowing. Accordingly, in a further aspect, the present invention provides a chewable, optionally effervescent, tablet comprising 1000/125mg amoxycillin and clavulanate.

Chewable tablets may be prepared by combining granulates as hereinbefore described with extra-granular excipients conventionally used to form a chewable base, for instance, mannitol, sorbitol, dextrose, fructose, or lactose, alone or in combination, preferably present in from 10 to 30% based on the weight of the final tablet, more preferably from 15 to 25%. The tablets may also contain conventional lubricants such as magnesium stearate, sweetening agents such as aspartame or sodium saccharin and flavouring and colouring agents. A disintegrating agent may also be incorporated as an extragranular excipient, to give the patient the option of dispersing the tablet in a small amount of water prior to administration. Suitable disintegrating agents include cellulose-based products such microfine cellulose, microcrystalline cellulose or hydroxypropyl cellulose as well as sodium strach glycollate and CLPVP. The disintegrant is preferably present in from 5 to 30%, more preferably from 5 to 15%, based on the weight of the final tablet.

Preferably, the chewable tablet is also provided with an effervescent couple. Effervescent chewable tablet formulations comprising 250/125 or 250/62.5mg amoxycillin/clavulanate have previously been described in EP 0 396 335 A1 (Beecham Group plc). Suitable such effervescent couples are well known in the art and typically comprise comprise a solid acid and an alkali metal carbonate or bicarbonate which generates carbon dioxide on contact with water, for instance citric acid, monosodium citrate or sodium hydrogen citrate and sodium (bi)carbonate. Other pharmaceutically acceptable acid/alkaline or alkaline earth metal carbonate mixtures may also be used, for instance tartaric, adipic, fumaric or malic acids and sodium, potassium or calcium (bi)carbonates or sodium glycine carbonate. Preferably, the two components are employed in a chemical molecular equivalent basis in the range 4:3 to 1:3, more preferably about 2:3 (acidic:basic component).

Preferred couples comprises citric acid/sodium bicarbonate and monosodium citrate/sodium bicarbonate, in particular monosodium citrate (56.04% w/w) and sodium bicarbonate(43.96% w/w). Preferably, this is provided as granules prepared from anhydrous powdered monosodium citrate and powdered sodium bicarbonate and compacted together by roller compaction, according to the process described in WO 97/02014 (SmithKline Beecham Laboratoires Pharmaceutiques). Suitable grades of anhydrous powdered monosodium citrate comprise particles which are substantially (i.e. about more than 90%) within the range 0 to 500 microns, preferably 355 microns, more preferably 0 to 250 microns. Suitable grades are available from Roche (monosodium citrate anhydrous powder, maximum of 5%w/w with grain size >0.250mm), Boehringer Ingelheim (monosodium zitrate wasserfrei Art-Nr 661 511, minimum of 90%w/w with grain size < 0.150mm), Jungbunzlauer (maximum of 5%w/w with grain size >0.355mm) and Haarmann & Reimer Corp. (maximum of 1% with grain size >0.500mm). Suitable grades of powdered sodium bicarbonate comprise particles

which are substantially (i.e. about more than 90%) within the range of 0 to 500 microns, preferably 270 microns, more preferably 0 to 130 microns. Suitable grades of powdered sodium bicarbonate are available from Solvay, for instance the grades 0 to 13 (particle size, by sieving method: >0.16mm max: 15g/kg) and extra-fine (particle size by sieving method: >0.125mm max; 20g/kg).

Preferably, the couple is present in from 5 to 30% of the final tablet weight, more preferably, from 5 to 15%, typically about 10%.

The granulates hereinbefore described may also be used for preparing other pharmaceutical formulations, in addition to tablets. For instance, they may be supplied as a sachet product containing a free-flowing granulated formulation in a suitable unit dose, for reconstituting in water to form an syrup formulation immediately prior to use, for example for administration to small children. Such free-flowing granulated formulation may comprise further excipients such as sweetening agents, thickeners, preservatives such as sodium benzoate and buffers such as sodium citrate.

Preferred sachets comprise:

amoxycillin granulates which consist essentially of amoxycillin trihydrate present in about 97wt% and CLPVP present in about 3wt%;
amoxycillin and potassium clavulanate granulates which comprise a 2:1 ratio of amoxycillin to clavulanate; silica gel present in about 10wt% of potassium clavulanate and CLPVP present in about 3wt% of amoxycillin trihydrate; and
extra granular excipients including silica gel (preferably 5 to 20%, more preferably about 10 to 15%), and flavour (preferably 2 to 10%, more preferably 3 to 8%).

Such sachets may preferably be provided as 'sugar-free' formulations, comprising, as an artificial sweetening agent such as aspartame, rather than sugar. Preferably, such formulations comprise preferably 1 to 10%, more preferably about 1 to 5% aspartame.

Such sachet formulations are of reduced weight in comparison to existing sachet formulations comprising corresponding weights of amoxycillin and clavulanate and therefore may be provided in smaller sachets, saving on packaging costs.

Further suitable formulations include encapsulated formulations which may optionally include an extra-granular lubricant, which if present is preferably in an amount of less than 0.5% by weight of the granulates, being contained within a pharmaceutical capsule. The pharmaceutical capsule may be an entirely conventional capsule, capable of dissolving in the stomach to release its contents, for example made of gelatine.

The sachet and encapsulated formulations described above preferably contain unit doses of amoxycillin, for instance sachets comprising nominally 125/31.25, 250/31.25, 250/62.5, 500/125, 500/62.5, 875/125 or 1000/125mg of amoxycillin/clavulanate.

Other suitable formulations include formulations for paediatric use which are reconstituted into aqueous suspensions prior to use. Such formulations may comprise further extragranular excipients such as, for example, a dessicating agent, for instance silica gel or dried maltodextrin, thickeners such as xanthan gum, carboxymethylcellulose sodium, silica gel, and hydroxypropylmethyl cellulose, preservatives such as sodium benzoate, sweetening agents such as aspartame, acesulfamate potassium, and sodium saccharin, lubricants such as magesium stearate, glidants such as colloidal silicon dioxide, and flavouring agents such as fruit flavour(s). Such formulations are found to be less powdery and so donot coat the inside of the bottle containing them , thereby enhancing their asethetic attributes. Further more, preferred formulations have a lower bulk so can be provided in a smaller container. Formulations are provided comprising an appropriate quantity of amoxycillin and clavulanate to be reconstituted into aqueous solution, for instance 100/12.5mg/ml in 30ml, 100/12.5mg/ml in 60ml, 125/31.25mg/5ml in 60 to 80ml and 250/62.5mg/5ml in 60 to 80ml.

In a further aspect, the present invention provides a process for preparing the formulations of the present invention hereinbefore defined which process comprises blending together a first set of granulates comprising amoxycillin and clavulanate with a second set of granulates comprising amoxycillin and no clavulanate in an appropriate ratio and then further processing to obtain the final desired formulation, for instance, compressing into tablets, filling into sachets, bottles or capsules.

It will be readily appreciated that a range of different formulations with differing ratios of amoxycillin to clavulanate may be readily be prepared by utilising as a common intermediate granulates comprising a given ratio of amoxycillin and clavulanate, for instance 2:1, and then adding in differing relative amounts of granulates comprising amoxycillin alone, to provide the different final ratios.

It will be further appreciated that the use of granulates comprising silica gel and silica gel as an extra-granular excipient, rather than microfine cellulose allows the development of a range of diverse formulation types with fewer different types of excipients, thereby further reducing the complexity of the manufacturing process.

Preferably, the amoxycillin and clavulanate, if present, (drug substance) is milled and sieved to achieve the desired particle size range. Preferably, the intra-granular disintegrant is also milled and sieved to a suitable particle size, for example, in the case of CLPVP, about 30μ, but particle size does not appear to be critical. Prepared drug substance and intra-granular excipients are then blended together in a dry state, and compacted under pressure. This may be by conventional dry compaction means, for example pressing, rolling, slugging extrusion etc, and a suitable pressure for the compaction process is 30-200KN, for instance 70-100, preferably, 80-90kN for amoxycillin granulates and 50-80, preferably, 60-70kN for amoxycillin/clavulanate granulates. The above-described granulate formulations are particularly suited to formation by roller compaction. The use of roller compaction to prepare granules comprising amoxycillin and potassium clavulanate is described in WO 92/19227 and WO 95/28927 (both to SmithKline Beecham).

Potassium clavulanate is known to be highly sensitive to moisture so that it is preferred that the preparation of the formulations of the invention is carried out under conditions of low humidity, for instance less than 30% RH, more preferably less than 20% RH, ideally 7-10% RH.

For further processing into tablets, it may be necessary to mill and sieve the granulates so as to achieve a suitable size fraction of the granulate. Compression into tablets may be carried out in a conventional manner, for instance on a conventional tabletting machine. As an optional further step the tablets may be coated as described above.

Formulations of this invention may be provided for treatment of bacterial infections generally, for example one or more of *inter alia* upper respiratory tract infections, lower respiratory tract infections, genito- urinary tract infections and skin and soft tissue infections.

Accordingly, in a further aspect, the present invention provides for the use of a first set of granulates comprising amoxycillin and clavulanate with a second set of granulates comprising amoxycillin and no clavulanate in the manufacture of a medicament for treating bacterial infections.

The invention will now be described, by way of example only. In these, the weights and % are the actual weights and % of amoxycillin trihydrate and potassium clavulanate, rather than the corresponding free acid equivalents.

In the following examples it is preferred to use amoxycillin trihydrate in which the Equilibrium Relative Humidity is in the range 10-20%, therebey allowing the relative amount of dessicant to be reduced to a minimum level.

### Example 1 - Amoxycillin granulate

Amoxycillin trihydrate was milled and sieved using an 0.04 or 0.027 inch (1.0 - 0.7 mm) aperture sieve, and was mixed for 15 minutes in a blender with dried cross-linked polyvinylpyrrolidinone (CLPVP) having a molecular weight of approximately 1 million and a density of 1.22 mg/cm$^3$ (Polyplasdone XL - Trade Mark), the mixture containing 3.4% of CLPVP by weight. The mixture was consolidated using a roller compacter at a controlled pressure of 50KN. The compacted flakes were granulated in a mill, or granulated through a sieve fitted with a 1mm mesh to obtain a suitable size fraction.

### Example 2 - Amoxycillin granulate

| Amoxycillin trihydrate | 97.087 % |
|---|---|
| CLPVP (Polyplasdone XL) | 2.913 |

Milled amoxycillin trihydrate and crospovidone (moisture <2%, sieved on a 1mm sieve) were separately weighed out and then introduced into a blender (amox first) for blending together, both operations being carried out in a controlled area (t° = 20 +/- 1°C, RH<30%). The resultant blend was discharged into double polyethylene bags in labelled containers and stored for subsequent roller compaction.

A Bepex roller compactor was fed with blend, maintaining a constant level in the feeding hopper, with the load cell set at 15kN and the compactor at a roller strength of 80-90kN. The resultant flakes were calibrated on oscillating granulator fitted with 1mm aperture screen. Uncompacted material which passed through a 1mm sieve was recycled. The granules were weighed and collected in double polyethylene bags and kegs. The roller compaction and calibration was performed in a controlled area (t° = 20 +/- 1°C, RH<30%)

### Example 3 - Amoxycillin granulate

Granulates were prepared using a procedure identical to that of example 1, comprising 97.12 weight % amoxycillin trihydrate together with 2.88 weight % sodium starch glycollate (as "Primogel") as intra-granular disintegrant.

### Example 4 - Amoxycillin/clavulanate granulate

Granulates comprising amoxycillin and clavulante in a ratio of 2:1 were prepared using a procedure identical to example 1, comprising:

| Amoxycillin trihydrate | 46.3% |
|---|---|
| Potassium clavulanate | 24.3 |
| Silica gel (Syloid AL-1) | 24.3 |
| CLPVP (Polyplasdone XL) (dried) | 1.38 |

Potassium clavulanate and silica gel are used as a 1:1 blend (based on the actual weight of potassium clavulanate).

### Example 5 - Amoxycillin/clavulanate granulate

Granulates comprising amoxycillin and clavulanate in a ratio of 2:1 and a lesser proportion of silica gel than in Example 4 were prepared using a procedure identical to example 1, comprising:

| Amoxycillin trihydrate | 63.3% |
|---|---|
| Potassium clavulanate | 31.7 |
| Silica gel (Syloid AL-1) | 3.1 |
| CLPVP (Polyplasdone XL) (dried) | 1.9 |

Milled amoxycillin, crospovidone (moisture <2%) and silica gel (sieved on 1 mm) and finally a 1:1 blend of amoxycillin trihydrate/potassium clavulante (sieved on 1 mm) were introduced in succession into a blender and blended together in a controlled area (t° = 20 +/- 1°C,RH<30%). The blend was then discharged into double polyethylene bags with desiccant in labelled containers for storage before roller compaction.

A Bepex roller compactor was fed with blend maintaining a constant level in the feeding hopper, with the load cell set at 15kN; and the compactor at a roller strength of 60-70kN. The resultant flakes were calibrated on oscillating granulator fitted with 1mm aperture screen. Uncompacted material which passed through a 1mm sieve was recycled. The granules were weighed and collected in double polyethylene bags and kegs. The roller compaction and calibration was performed in a controlled area (t° = 20 +/- 1°C, RH<30%)

### Example 6 - Reduced weight tablets

A reduced weight tablet comprising 500mg/62.5mg, 500mg/125mg, 875mg/125mg or 1g/125mg amoxycillin/clavulanate was prepared by combining granulates comprising amoxycillin and potassium clavulanate (Example 5) in a 2:1 ratio with granulates comprising amoxycillin (Example 2), to give an overall ratio of 8:1, 4:1, 7:1 or 8:1 respectively with extragranular excipients, in particular low substituted hydroxypropyl cellulose as the extragranular disintegrant, so that the overall composition was as follows:

| | 500/125 mg | 875/125 mg | 1g/125 mg |
|---|---|---|---|
| **Core** | mg | mg | mg |
| Amoxycillin trihydrate (100% of theory; equivalent to 500, 875mg or 1g Amox. 100%) | 573.87 | 1004.272 | 1147.74 |
| Potassium Clavulanate (100% of theory; equivalent to 125mg Clavulanic acid 100%) | 148.93 | 148.930 | 148.930 |
| Silica Gel[1] (intra-granular) | 14.89 | 14.890 | 14.890 |
| CLPVP (intra-granular) | 17.21 | 30.130 | 34.430 |
| Silica Gel[1] (extra-granular) | 8.3 | 14.000 | 16.000 |
| Low substituted hydroxypropyl cellulose[2] | 50.00 | 70.000 | 80.000 |

[1] Syloid AL-1

[2]: L-HPC LH-11 or equivalent;

(continued)

|  | 500/125 mg | 875/125 mg | 1g/125 mg |
|---|---|---|---|
| **Core** | **mg** | **mg** | **mg** |
| Colloidal silica[3] | 1.70 | 2.700 | 3.000 |
| Magnesium stearate | 3.00 | 4.600 | 5.300 |
| Total weight (core) | 817.9 | 1289.520 | 1450.29 |
|  |  |  |  |
| **Coating** |  |  |  |
| Hydroxypropyl methylcellulose 5cP | 6.93 | 10.925 | 12.288 |
| Hydroxypropyl methylcellulose 15cP | 2.31 | 3.642 | 4.096 |
| Polyethylene Glycol 4000 | 1.369 | 2.158 | 2.427 |
| Polyethylene Glycol 6000 | 1.369 | 2.158 | 2.427 |
| Titanium dioxide | 9.022 | 14.225 | 15.997 |
| Total weight of coating | 21 | 33.108 | 37.235 |
|  |  |  |  |
| Total weight of coated tablet | 838.9 | 1322.628 | 1487.525 |
|  | **500/62.5 mg** | **500/62.5 mg** | **500/62.5 mg** |
| **Core** | **mg** | **mg** | **mg** |
| Amoxycillin trihydrate (100% of theory; equivalent to 500, 875mg or 1g Amox. 100%) | 573.87 | 573.87 | 573.87 |
| Potassium Clavulanate (100% of theory; equivalent to 125mg Clavulanic acid 100%) | 74.46 | 74.46 | 74.46 |
| Silica Gel[1] (intra-granular) | 7.45 | 7.45 | 7.45 |
| CLPVP (intra-granular) | 17.21 | 17.21 | 17.21 |
| Silica Gel[1] (extra-granular) | 7.4 | 15.3 | 17.2 |
| Low substituted hydroxypropyl cellulose[2] | 44.5 | 61.3 | 78.4 |
| Colloidal silica[3] | 1.50 | 1.50 | 1.50 |
| Magnesium stearate | 2.7 | 2.8 | 2.8 |
| Total weight (core) | 729.09 | 753.89 | 772.99 |
|  |  |  |  |
| Coating (as above) | 19 | 19 | 19 |
| Total weight of coated tablet | 748.09 | 772.89 | 792.99 |

[1] Syloid AL-1

[2]: L-HPC LH-11 or equivalent;

[3]: Aerosil 200 or equivalent.

The manufacture of tablets was carried out in a controlled area (t° = 20 +/- 1°C, RH<30%). A compression mix was prepared by introducing into a blender Amoxycillin granulates; Amoxycillin/Clavulanate 2/1 granulates; silica gel ,L-HPC LH-11 and colloïdal silicon dioxide sieved on 0.5mm. These materials were blended together, Magnesium stearate (sieved on 0.5mm) was then added, followed by further blending. This mix was then compressed using concave oval punches to give tablets of the desired weight. Uncoated tablets were sampled every 5-15 mn during compression run and checked for hardness, thickness, mean weight, uniformity of mass and friability.

The uncoated tablet cores were then film coated with a coating suspension (15w/w% in water) on a suitable coating machine, rotating the cores and spraying the film coating suspension until theoretical quantity of film coat had been applied (WO 95/28927, SmithKline Beecham).

The film coated tablets were then stored in appropriate sealed containers.

For the 500/125mg tablet, the tablet has a total weight of about 817.9mg, including 63mg of extragranular excipients plus a coating of about 21mg. Amoxycillin trihydrate and potassium clavulanate account for about 89.6% of the core weight of the tablet. This may be contrasted with the present 500/125mg tablet which has a core weight of 1050mg of which amoxycillin trihydrate and potassium clavulanate account for about 69.3% and microcrystalline cellulose about 27%. The quantities may be halved to give a 250/62.5mg tablet

In the 875/125 mg, amoxycillin trihydrate and potassium clavulanate account for about 89.43% of the core weight of the tablet This can be contrasted with the current 875/125mg tablet which has a core weight of 1450mg, of which amoxycillin trihydrate and potassium clavulanate account for about 80% and microcrystalline cellulose about 15.6% (WO 95/28927, SmithKline Beecham).

In the 1g/125mg tablets, amoxycillin trihydrate and potassium clavulanate account for about 89.41% of the core weight of the tablet The quantities may be halved, to give a 500/62.5mg tablet.

## Example 7 - Reduced weight tablets

A reduced weight tablet comprising 500mg/125mg, 875mg/125mg or 1g/125mg amoxycillin/clavulanate was prepared by combining granulates comprising amoxycillin and potassium clavulanate (Example 5) in a 2:1 ratio with granulates comprising amoxycillin (Example 1), to give an overall ratio of 4:1, 7:1 or 8:1 respectively with extragranular excipients, in particular CLPVP as the extragranular disintegrant, so that the overall composition was as follows:

|  | 500/125 mg | 875/125 mg | 1g/125 mg |
|---|---|---|---|
| Core | mg | mg | mg |
| Amoxycillin trihydrate (based on 86.4% assay for amox; equivalent to 500, 875mg or 1g Amox. 100%) | 578.7 | 1012.7 | 1157.4 |
| Potassium Clavulanate (based on 82.5% assay for KCA; equivalent to 125mg Clavulanic acid 100%) | 151.5 | 151.5 | 151.5 |
| Silica Gel[1] (intra-granular) | 15.2 | 15.2 | 15.2 |
| CLPVP (intra-granular) | 17.4 | 30.4 | 34.7 |
| CLPVP (extra-granular) | 60 | 90 | 100 |
| Magnesium stearate | 3.00 | 4.50 | 5.0 |
| Total weight (core) | 825.8 | 1304.3 | 1463.8 |

[1] Syloid AL-1

A suitable solvent based coating coating for the cores of the above tablets is as follows:

| Dimethicone 200 | 0.04mg |
|---|---|
| Ethyl cellulose | 3.21 |
| Hydroxypropylmethyl cellulose 15cps | 12.8 |
| Titanium dioxide suspension | 11.34 |
| Diethyl phthalate | 3.78 |
| Total weight of coating | 31.17 |

## Example 8 - 500mg/125mg and 1g/125mg Sachets

Granulates are prepared using procedures identical to Examples 1 and 5. The granulates are then blended in the appropriate ratio with typical sachet excipients to produce a sachet formula comprising:

|  | 500/125 mg | 1g/125mg |
|---|---|---|
| Amoxycillin trihydrate 100% of theory (equivalent to 500mg or 1g amoxycillin 100%) | 573.87 mg | 1147.4 |
| Potassium Clavulanate 100% of theory (equivalent to 125mg Clavulanic acid 100%) | 148.93 | 148.93 |
| CLPVP (intra-granular) | 17.21 | 34.42 |

(continued)

|  | 500/125 mg | 1g/125mg |
|---|---|---|
| Silica gel[1] (intra-granular) | 14.89 | 14.89 |
| Silica gel[1] (extra-granular) | 65.00 | 130.00 |
| Aspartame | 15.00 | 30.00 |
| Peach-Lemon-Strawberry flavour | 30.00 | 60.0 |
| Total Weight | 864.90 | 1565.98 |

[1] Syloid AL-1

Sachets comprising 500/62.5mg and 250.31.25mg may be prepared using *pro rata* amounts based on the 1g/125mg formulation.

### Example 9 - 250/62.5, 500/125 and 875/125mg Sachets

Granulates are prepared using procedures identical to Examples 1 and 5. The granulates are then blended in the appropriate ratio with typical sachet excipients to produce a sachet formula comprising:

|  | 250/62.5 mg | 500/125 mg | 875/125 mg |
|---|---|---|---|
| Amoxycillin trihydrate (based on 86.4% assay for amox, equivalent to 250, 500 and 875 mg amoxycillin 100%) | 289.35 | 573.87 mg | 1147.4 |
| Potassium Clavulanate (based on 82.5% assay for K clav, equivalent to 62.5 and 125mg Clavulanic acid 100%) | 75.75 | 148.93 | 148.93 |
| CLPVP (infra-granular) | 8.7 | 17.4 | 30.4 |
| Silica gel[1] (intra-granular) | 7.6 | 15.2 | 15.2 |
| Silica gel[1] (extra-granular) | 167.5 | 335 | 335 |
| Aspartame | 6 | 12.00 | 12.00 |
| Peach-Lemon-Strawberry flavour | 107 | 214 | 214 |
| Microcrystalline cellulose | 213.1 | 426.2 | 229.2 |

[1]Syloid AL-1

### Example 10 - 100mg/12.5mg/ml suspension - 30ml

Granulates are prepared using procedures identical to Examples 1 and 5. The granulates are then blended in the appropriate ratio with typical suspension excipients to produce a sachet formula comprising:

| | |
|---|---|
| Amoxycillin trihydrate 100% of theory (equivalent to 3000mg Amoxycillin 100%) | 3443.22 mg |
| Potassium Clavulanate 100% of theory (equivalent to 375mg Clavulanic acid 100%) | 446.79 |
| CLPVP (intra-granular) | 103.29 |
| Silica gel[1] (intra-granular) | 44.68 |
| Silica gel[1] (extra-granular) | 500.00 |
| Xanthan gum | 25.20 |
| Carboxymethylcellulose sodium salt | 250.00 |
| Sodium benzoate | 51.00 |
| Hydrophobic colloidal silica | 15.00 |
| Aspartame | 96.00 |
| Magnesium stearate | 10.00 |
| Strawberry flavour | 150.00 |
| Total weight | 5135.50 mg |

[1] Syloid AL-1

The formulation is made up into 30ml of aqueous solution immediately prior to first use. The same formulation may also be made up into 60ml aqeous solution.

**Example 11 - Chewable effervescent tablet (1g/125mg)**

| | mg |
|---|---|
| Amoxycillin trihydrate (equivalent to amoxycillin 1000.0) | 1147.74 |
| Potassium clavulanate (equivalent to clavulanic acid 125.0) | 148.93 |
| CLPVP | 34.43 |
| Silica gel[1] (intragranular) | 14.89 |
| Silica gel[1] (extragranular) | 120.00 |
| Monosodium citrate | 156.91 |
| Sodium bicarbonate | 123.09 |
| Microcrystalline cellulose[2] | 280.00 |
| Flavour | 90.00 |
| Aspartame | 40.00 |
| Magnesium stearate | 30.00 |
| Mannitol DC | 614.01 |
| Total weight | 2,800 |

[1] Crospovidone
[2] Syloid AL-1
[3] Avicel PH112

**Example 12 - Conventional 500/125mg tablet**

| Core | |
|---|---|
| Amoxycillin trihydrate (equivalent to amoxycillin 500.0) | 578.87 |
| Potassium clavulanate (equivalent to clavulanic acid 125.0) | 148.93 |
| Anhydrous colloidal silica | 10.50 |
| Sodium starch glycollate[1] | 21.00 |
| Magnesium stearate | 7.27 |
| Microcrystalline cellulose | qs 1050 |
| **Coating** | |
| Methylhydroxypropylcellulose | 11.88 |
| Polyethylene glycol 4000 | 1.76 |
| Polyethylene glycol 6000 | 1.76 |
| Titanium dioxide | 11.60 |
| Dimethicone | 0.14 |
| Coating | 27.14 |
| Total | 1077mg |

[1] (Primojel/Explotab)

**Example 13 - 500/125mg tablet**

| Core | |
|---|---|
| Amoxycillin trihydrate (equivalent to amoxycillin 500.0, based on 86% potency) | 581.4 |
| Potassium clavulanate (equivalent to clavulanic acid 62.5, based on 82% potency) | 76.2 |
| Colloidal silicon dioxide | 8.2 |

(continued)

| Core | |
|---|---|
| Sodium starch glycollate | 16.4 |
| Microcrystalline cellulose | 129.1 |
| Magnesium stearate | 5.7 |
| Weight | $\overline{817.0}$ |
| **Coating** | |
| as in Example 6 | 24.5 |
| Total | 841.5mg |

Amocycillin, clavulanate source (either as a 1:1 blend of clav:amox or a 1:1 blend of clav and microcrystalline cellulose), most of the microcrystalline cellulose and a portion of the magnesium stearate were blended together. This blend was then roller-compacted and milled to form granules. These granules were blended with the sodium starch glycollate, colloidal silicon dioxide, the remaining microcrystalline cellulose (approx 38.55 of the total amount) and the magnesium stearate, and the blend then compressed into tablet cores. In a final step, the cores were coated with an aqueous suspension of the coating.

The weights and relative proportions of the components of the foregoing examples 1 to 13 may be varied about the figures listed but preferably are within ±10%, more preferably within ±5%, preferably within ±2.5%. Overages of active ingredients may be used if necessary and justified.

It will be appreciated that the the foregoing formulations may also be produced using a single set of granules comprising amoxycillin and clavulanate in the final ratio of 4:1, 7:1, or 8:1 rather than the two sets described, using intra- and extra-granular excipients in the same proportions. The present invention covers all such formulations.

**Claims**

1.   A granulate comprising amoxycillin trihydrate and potassium clavulanate in a ratio of 1:1, 2:1 or 4:1, wherein this ratio refers to the weight of parent compound amoxycillin or clavulanic acid, and an intragranular diluent present in from 5 to 25% by weight of potassium clavulanate.

2.   A granulate as claimed in claim 1 wherein the intragranular diluent is present from 5 to 15% by weight of potassium clavulanate.

3.   A granulate as claimed in claim 1 or 2 wherein the intragranular diluent is silica gel.

4.   A granulate as claimed in any of claims 1 to 3 which further comprises an intragranular disintegrant present in from 1.25 to 3.5% by weight of the granulate.

5.   A granulate as claimed in claim 4 wherein the intragranular disintegrant is sodium starch glycollate or cross-linked N-vinyl-2-pyrrolidinone.

6.   A granulate as claimed in any of claims 1 to 5 which further comprises an intragranular lubricant present in from 0 to 0.35% by weight of the granulate.

7.   A granulate as claimed in claim 6 wherein the intragranular lubricant is magnesium stearate.

8.   A granulate as claimed in any of claims 1 to 7 wherein the equilibrium relative humidity of the amoxycillin trihydrate is from 10 to 20%.

9.   A pharmaceutical formulation comprising granulates as defined in any of claims 1 to 8 and granulates comprising amoxycillin trihydrate (and no clavulanate) such that the overall ratio of amoxycillin trihydrate to potassium clavulanate is in a ratio of n: 1 where n is a number from 2 to16 and the ratio refers to the weight of parent compound amoxycillin or clavulanic acid.

10. A formulation as claimed in claim 9 wherein the overall ratio of amoxycillin trihydrate to potassium clavulanate is selected from 2:1, 4:1, 6:1, 7:1, 8:1 or 14:1 and the ratio refers to the weight of parent compound amoxycillin or clavulanic acid.

11. A formulation as claimed in claims 9 or 10 further comprising an extra-granular dessicant present in an amount up to 5% by weight of the formulation.

12. A formulation as claimed in claim 11 wherein the extra-granular dessicant is silica gel.

13. A formulation as claimed in any of claims 9 to 12 comprising amoxycillin trihydrate and potassium clavulanate in a ratio of n:1, where n is a number from 2 to 16 and the ratio refers to the weight of parent compound amoxycillin or clavulanic acid, and which comprises granulates consisting essentially of amoxycillin trihydrate and potassium clavulanate, cross-linked N-vinyl-2-pyrrolidinone (as an intragranular disintegrant) present in about 3wt% of amoxycillin trihydrate and silica gel (as an intragranular diluent/dessicant) present in about 10wt% of potassium clavulanate.

14. A formulation as claimed in any of claims 9 to 13 provided for paediatric use which is reconstituted into an aqueous suspension prior to use.

15. A paediatric formulation as claimed in claim 14 which further comprises extragranular excipients selected from a dessicating agent, thickeners, preservatives, sweetening agents, lubricants, glidants, and flavouring agents.

16. A paediatric formulation as claimed in claim 15 wherein the thickener is selected from xanthan gum, carboxymethylcellulose sodium, silica gel or hydroxpropylmethyl cellulose.

17. A paediatric formulation as claimed in claims 15 or 16 wherein the flavouring agent is a fruit flavour.

18. A formulation as claimed in any of claims 9 to 13 provided as a sachet product and containing free-flowing granulates in a suitable unit dose, for reconstituting in water to form a syrup formulation immediately prior to use.

19. A sachet product as claimed in claim 18 which further comprises excipients selected from sweetening agents, thickeners, preservatives and buffers.

20. A formulation as claimed in any of claims 9 to 13 provided as a tablet, which further comprises an extra-granular disintegrant.

21. A tablet formulation as claimed in claim 20 wherein the extra-granular disintegrant is low-substituted hydroxypropylcellulose.

22. A tablet formulation as claimed in claims 20 or 21 which further comprises an extra-granular lubricant.

23. A tablet formulation as claimed in claim 22 wherein the extra-granular lubricant is magnesium stearate.

24. A tablet formulation as claimed in any of claims 20 to 23 which comprises amoxycillin granulates which consist essentially of amoxycillin trihydrate present in about 97wt% and cross-linked N-vinyl-2-pyrrolidinone present in about 3wt%; amoxycillin and potassium clavulanate granulates which comprise a 2:1 ratio of amoxycillin trihydrate to potassium clavulanate wherein the ratio refers to the weight of parent compound amoxycillin or clavulanic acid; silica gel present in about 10wt% of potassium clavulanate and about 3wt% of cross-linked N-vinyl-2-pyrrolidinone; and extra-granular excipients including: silica gel (1.0 to 1.2% by weight of the tablet) ; low-substituted hydroxypropylcellulose (5 to 6% by weight of the tablet); colloidal silica (0.1 to 0.3% by weight of the tablet); and magnesium stearate (0.3 to 0.4% by weight of the tablet); or cross-linked N-vinyl-2-pyrrolidinone (5 to 8% by weight of the tablet); and magnesium stearate (0.3 to 0.4% by weight of the tablet).

25. A formulation as claimed in any of claims 9 to 13 provided as a chewable tablet, which further comprises an extra-granular excipient, present in from 10 to 30% based on the weight of the final tablet, used to form a chewable base.

26. A tablet formulation as claimed in claim 25 wherein the extra-granular excipient is selected from mannitol, sorbitol, dextrose, fructose, or lactose, alone or in combination.

27. A tablet formulation as claimed in claims 25 or 26 which further comprises a disintegrating agent present in from 5 to 30% based on the weight of the final tablet.

28. A tablet formulation as claimed in claim 27 wherein the disintegrating agent is selected from microfine cellulose, microcrystalline cellulose, hydroxypropyl cellulose, sodium starch glycollate or cross-linked N-vinyl-2-pyrrolidinone.

29. A tablet formulation as claimed in claims 25 or 26 which further comprises an effervescent couple, present in from 5 to 30% based on the weight of the final tablet.

30. A tablet formulation as claimed in claim 29 wherein the effervescent couple comprises a solid acid selected from citric acid, monosodium citrate, sodium hydrogen citrate, tartaric acid, adipic acid, fumaric acid or malic acid, and an alkali or alkaline earth metal carbonate or bicarbonate selected from sodium (bi)carbonate, potassium (bi) carbonate, calcium (bi)carbonate or sodium glycine carbonate.

31. A tablet formulation as claimed in any of claims 20 to 30 which comprises amoxycillin trihydrate, potassium clavulanate and pharmaceutically acceptable excipients, wherein the amoxycillin trihydrate is present in an amount equivalent to 500mg of amoxycillin (free acid) and the potassium clavulanate is present in an amount equivalent to 62.5mg of clavulanic acid.

32. A sachet formulation for suspension according to claim 18 in which the weights and relative proportions of the components are:

| | |
|---|---|
| Amoxycillin trihydrate 100% of theory (equivalent to 3000mg Amoxycillin 100%) | 3443.22 mg |
| Potassium Clavulanate 100% of theory | 446.79 mg |
| (equivalent to 375mg Clavulanic acid 100%) cross-linked N-vinyl-2-pyrrolidinone (CLPVP) (intra-granular) | 103.29 mg |
| Silica gel (intra-granular) (Syloid AL) | 44.68 mg |
| Silica gel (extra-granular) (Syloid AL) | 500.00 mg |
| Xanthan gum | 25.20 mg |
| Carboxymethylcellulose sodium salt | 250.00 mg |
| Sodium benzoate | 51.00 mg |
| Hydrophobic colloidal silica | 15.00 mg |
| Aspartame | 96.00 mg |
| Magnesium stearate | 10.00 mg |
| Strawberry flavour | 150.00 mg |

wherein the weights and relative proportions of the components may be varied about the figures listed but are within ± 10%.

33. A process for preparing a formulation according to any of claims 9 to 32 which comprises blending together a first set of granulates according to claims 1-8 comprising amoxycillin trihydrate and potassium clavulanate with a second set of granulates comprising amoxycillin trihydrate and no clavulanate in an appropriate ratio with other excipients and then, if necessary and desired, further processing the blend to obtain the final desired formulation.

**Patentansprüche**

1. Granulat, das Amoxycillintrihydrat und Kaliumclavulanat in einem Verhältnis von 1:1, 2:1 oder 4:1, worin dieses Verhältnis das Gewicht der Stammverbindung Amoxycillin oder Clavulansäure bezeichnet, und ein intragranulares Verdünnungsmittel in einer Menge von 5 bis 25 Gew.% von Kaliumclavulanat umfasst.

2. Granulat gemäss Anspruch 1, worin das intragranulare Verdünnungsmittel in einer Menge von 5 bis 15 Gew.% von Kaliumclavulanat vorhanden ist.

3. Granulat gemäss Anspruch 1 oder 2, worin das intragranulare Verdünnungsmittel Kieselgel ist.

**4.** Granulat gemäss einem der Ansprüche 1 bis 3, das ferner ein intragranulares Tablettensprengmittel in einer Menge von 1,25 bis 3,5 Gew.% des Granulats umfasst.

**5.** Granulat gemäss Anspruch 4, worin das intragranulare Tablettensprengmittel Natriumstärkeglykolat oder vernetztes N-Vinyl-2-pyrrolidinon ist.

**6.** Granulat gemäss einem der Ansprüche 1 bis 5, das ferner ein intragranulares Schmiermittel in einer Menge von 0 bis 0,35 Gew.% des Granulats umfasst.

**7.** Granulat gemäss Anspruch 6, worin das intragranulare Schmiermittel Magnesiumstearat ist.

**8.** Granulat gemäss einem der Ansprüche 1 bis 7, worin die relative Gleichgewichtsfeuchtigkeit des Amoxycillintrihydrats 10 bis 20 % ist.

**9.** Pharmazeutische Formulierung, die Granulate wie in einem der Ansprüche 1 bis 8 definiert und Granulate umfasst, die Amoxycillintrihydrat (und kein Clavulanat) umfassen, so dass das Gesamtverhältnis von Amoxycillintrihydrat zu Kaliumclavulanat in einem Verhältnis von n:1 ist, worin n eine Zahl von 2 bis 16 ist und das Verhältnis das Gewicht der Stammverbindung Amoxycillin oder Clavulansäure bezeichnet.

**10.** Formulierung gemäss Anspruch 9, worin das Gesamtverhältnis von Amoxycillintrihydrat zu Kaliumclavulanat aus 2:1, 4:1, 6:1, 7:1, 8:1 oder 14:1 ausgewählt ist und das Verhältnis das Gewicht der Stammverbindung Amoxycillin oder Clavulansäure bezeichnet.

**11.** Formulierung gemäss Anspruch 9 oder 10, die ferner ein extragranulares Trockenmittel in einer Menge von bis zu 5 Gew.% der Formulierung umfasst.

**12.** Formulierung gemäss Anspruch 11, worin das extragranulare Trockenmittel Kieselgel ist.

**13.** Formulierung gemäss einem der Ansprüche 9 bis 12, die Amoxycillintrihydrat und Kaliumclavulanat in einem Verhältnis von n:1 umfasst, worin n eine Zahl von 2 bis 16 ist und das Verhältnis das Gewicht von Stammverbindung Amoxycillin oder Clavulansäure bezeichnet, und die Granulate, die im wesentlichen aus Amoxycillintrihydrat und Kaliumclavulanat bestehen, vernetztes N-Vinyl-2-pyrrolidinon (als intragranulares Tablettensprengmittel) in einer Menge von ca. 3 Gew.% von Amoxycillintrihydrat und Kieselgel (als intragranulares Verdünnungsmittel/Trockenmittel) in einer Menge von ca. 10 Gew.% von Kaliumclavulanat umfasst.

**14.** Formulierung gemäss einem der Ansprüche 9 bis 13, vorgesehen zur pädiatrischen Verwendung, die vor der Verwendung zu einer wässrigen Suspension rekonstituiert wird.

**15.** Pädiatrische Formulierung gemäss Anspruch 14, die ferner extragranulare Hilfsstoffe umfasst, ausgewählt aus Trockenmittel, Verdickungsmitteln, Konservierungsmitteln, Süssungsmitteln, Schmiermitteln, Fliessregulierungsmitteln und Geschmacksstoffen.

**16.** Pädiatrische Formulierung gemäss Anspruch 15, worin das Verdickungsmittel aus Xanthangummi, Carboxymethylcellulosenatrium, Kieselgel oder Hydroxypropylmethylcellulose ausgewählt ist.

**17.** Pädiatrische Formulierung gemäss Anspruch 15 oder 16, worin der Geschmacksstoff ein Fruchtgeschmack ist.

**18.** Formulierung gemäss einem der Ansprüche 9 bis 13, die als Sachet-Produkt bereitgestellt wird und freifliessende Granulate in einer geeigneten Einheitsdosis enthält, zur Rekonstituierung in Wasser zur Bildung einer Sirupformulierung unmittelbar vor der Verwendung.

**19.** Sachet-Produkt gemäss Anspruch 18, das ferner Hilfsstoffe umfasst, ausgewählt aus Süssungsmitteln, Verdickungsmitteln, Konservierungsmitteln und Puffern.

**20.** Formulierung gemäss einem der Ansprüche 9 bis 13, die als Tablette bereitgestellt wird, die ferner ein extragranulares Tablettensprengmittel umfasst.

**21.** Tablettenformulierung gemäss Anspruch 20, worin das extragranulare Tablettensprengmittel niedrigsubstituierte Hydroxypropylcellulose ist.

22. Tablettenformulierung gemäss Anspruch 20 oder 21, die ferner ein extragranulares Schmiermittel umfasst.

23. Tablettenformulierung gemäss Anspruch 22, worin das extragranulare Schmiermittel Magnesiumstearat ist.

24. Tablettenformulierung gemäss einem der Ansprüche 20 bis 23, die Amoxycillingranulate, die im wesentlichen aus Amoxycillintrihydrat in einer Menge von ca. 97 Gew.% und vernetztem N-Vinyl-2-pyrrolidinon in einer Menge von ca. 3 Gew.% bestehen; Amoxycillin- und Kaliumclavulanat-Granulate, die ein 2:1-Verhältnis von Amoxycillintrihydrat zu Kaliumclavulanat, worin das Verhältnis das Gewicht von Stammverbindung Amoxycillin oder Clavulansäure bezeichnet; Kieselgel in einer Menge von ca. 10 Gew.% von Kaliumclavulanat und ca. 3 Gew.% vernetztes N-Vinyl-2-pyrrolidinon umfassen; und extragranulare Hilfsstoffe umfasst, einschliesslich: Kieselgel (1,0 bis 1,2 Gew.% der Tablette); niedrigsubstituierte Hydroxypropylcellulose (5 bis 6 Gew.% der Tablette); kolloidale Kieselerde (0,1 bis 0,3 Gew.% der Tablette); und Magnesiumstearat (0,3 bis 0,4 Gew.% der Tablette); oder vernetztes N-Vinyl-2-pyrrolidinon (5 bis 8 Gew.% der Tablette); und Magnesiumstearat (0,3 bis 0,4 Gew.% der Tablette).

25. Formulierung gemäss einem der Ansprüche 9 bis 13, die als kaubare Tablette bereitgestellt wird, die ferner einen extragranularen Hilfsstoff in einer Menge von 10 bis 30 % auf Basis des Gewichts der fertigen Tablette umfasst, verwendet zur Bildung einer kaubaren Basis.

26. Tablettenformulierung gemäss Anspruch 25, worin der extragranulare Hilfsstoff aus Mannit, Sorbit, Dextrose, Fructose oder Lactose, allein oder in Kombination, ausgewählt ist.

27. Tablettenformulierung gemäss Anspruch 25 oder 26, die ferner ein Tablettensprengmittel in einer Menge von 5 bis 30 % auf Basis des Gewichts der fertigen Tablette umfasst.

28. Tablettenformulierung gemäss Anspruch 27, worin das Tablettensprengmittel aus mikrofeiner Cellulose, mikrokristalliner Cellulose, Hydroxypropylcellulose, Natriumstärkeglykolat oder vernetztem N-Vinyl-2-pyrrolidinon ausgewählt ist.

29. Tablettenformulierung gemäss Anspruch 25 oder 26, die ferner eine Brausekombination in einer Menge von 5 bis 30 % auf Basis des Gewichts der fertigen Tablette umfasst.

30. Tablettenformulierung gemäss Anspruch 29, worin die Brausekombination eine feste Säure, ausgewählt aus Zitronensäure, Mononatriumcitrat, Natriumhydrogencitrat, Weinsäure, Adipinsäure, Fumarsäure oder Äpfelsäure, und ein Alkali- oder Erdalkalimetallcarbonat oder -bicarbonat, ausgewählt aus Natrium(bi)carbonat, Kalium(bi)carbonat, Calcium(bi)carbonat oder Natriumglycincarbonat, umfasst.

31. Tablettenformulierung gemäss einem der Ansprüche 20 bis 30, die Amoxycillintrihydrat, Kaliumclavulanat und pharmazeutisch akzeptable Hilfsstoffe umfasst, worin das Amoxycillintrihydrat in einer Menge entsprechend 500 mg Amoxycillin (freie Säure) und das Kaliumclavulanat in einer Menge entsprechend 62,5 mg Clavulansäure vorhanden ist.

32. Sachet-Formulierung zur Suspension gemäss Anspruch 18, worin die Gewichte und relativen Anteile der Komponenten sind:

| | |
|---|---|
| Amoxycillintrihydrat (100 % der Theorie; entsprechend 3.000 mg Amoxycillin 100 %) | 3.443,22 mg |
| Kaliumclavulanat (100 % der Theorie; entsprechend 375 mg Clavulansäure 100 %) | 446,79 mg |
| vernetztes N-Vinyl-2-pyrrolidinon (CLPVP) (intragranular) | 103,29 mg |
| Kieselgel (intragranular) (Syloid AL) | 44,68 mg |
| Kieselgel (extragranular) (Syloid AL) | 500,00 mg |
| Xanthangummi | 25,20 mg |
| Carboxymethylcellulose-Natriumsalz | 250,00 mg |
| Natriumbenzoat | 51,00 mg |
| Hydrophobe kolloidale Kieselerde | 15,00 mg |
| Aspartam | 96,00 mg |

(fortgesetzt)

| Magnesiumstearat | 10,00 mg |
| Erdbeergeschmack | 150,00 mg |

worin die Gewichte und relativen Anteile der Komponenten um die aufgeführten Zahlen herum variiert werden können, aber innerhalb ± 10 % sind.

**33.** Verfahren zur Herstellung einer Formulierung gemäss einem der Ansprüche 9 bis 32, welches das Zusammenmischen eines ersten Satzes von Granulaten gemäss Ansprüchen 1 bis 8, die Amoxycillintrihydrat und Kaliumclavulanat umfassen, mit einem zweiten Satz von Granulaten, die Amoxycillintrihydrat und kein Clavulanat umfassen, in einem geeigneten Verhältnis mit anderen Hilfsstoffen und dann, falls notwendig und gewünscht, das weitere Verarbeiten der Mischung zum Erhalt der fertigen gewünschten Formulierung umfasst.

**Revendications**

**1.** Granulé comprenant du trihydrate d'amoxycilline et du clavulanate de potassium selon un rapport de 1:1, 2:1 ou 4:1, dans lequel ce rapport fait référence au poids du composé parent amoxycilline ou acide clavulanique, et un diluant intragranulaire présent dans une proportion de 5 à 25 % en poids de clavulanate de potassium.

**2.** Granulé selon la revendication 1, dans lequel le diluant intragranulaire est présent dans une proportion de 5 à 15 % en poids de clavulanate de potassium.

**3.** Granulé selon la revendication 1 ou 2, dans lequel le diluant intragranulaire est le gel de silice.

**4.** Granulé selon l'une quelconque des revendications 1 à 3, qui comprend en outre un délitant intragranulaire présent dans une proportion de 1,25 à 3,5 % en poids du granulé.

**5.** Granulé selon la revendication 4, dans lequel le délitant intragranulaire est le glycolate d'amidon sodique ou la N-vinyl-2-pyrrolidinone réticulée.

**6.** Granulé selon l'une quelconque des revendications 1 à 5, qui comprend en outre un lubrifiant intragranulaire présent dans une proportion de 0 à 0,35 % en poids du granulé.

**7.** Granulé selon la revendication 6, dans lequel le lubrifiant intragranulaire est le stéarate de magnésium.

**8.** Granulé selon l'une quelconque des revendications 1 à 7, dans lequel l'humidité relative d'équilibre du trihydrate d'amoxycilline est de 10 à 20 %.

**9.** Formulation pharmaceutique comprenant des granulés selon l'une quelconque des revendications 1 à 8 et des granulés comprenant du trihydrate d'amoxycilline (et pas de clavulanate) de sorte que le rapport global trihydrate d'amoxycilline/clavulanate de potassium se situe dans un rapport de n:1, où n est un nombre allant de 2 à 16 et le rapport fait référence au poids du composé parent amoxycilline ou acide clavulanique.

**10.** Formulation selon la revendication 9, dans laquelle le rapport global trihydrate d'amoxycilline/clavulanate de potassium est choisi parmi 2:1, 4:1, 6:1, 7:1, 8:1 ou 14:1 et le rapport fait référence au poids du composé parent amoxycilline ou acide clavulanique.

**11.** Formulation selon les revendications 9 ou 10, comprenant en outre un dessicant extragranulaire présent dans une quantité allant jusqu'à 5 % en poids de la formulation.

**12.** Formulation selon la revendication 11, dans laquelle le dessicant extragranulaire est le gel de silice.

**13.** Formulation selon l'une quelconque des revendications 9 à 12, comprenant du trihydrate d'amoxycilline et du clavulanate de potassium selon un rapport de n:1, où n est un nombre allant de 2 à 16 et le rapport fait référence au poids du composé parent amoxycilline ou acide clavulanique, et qui comprend des granulés consistant essentiellement en du trihydrate d'amoxycilline et du clavulanate de potassium, de la N-vinyl-2-pyrrolidinone réticulée (en tant que délitant intragranulaire) présente dans environ 3 % en poids du trihydrate d'amoxycilline et du gel de silice (sous forme de diluant/dessicant intragranulaire) présent dans environ 10 % en poids du clavulanate de potassium.

14. Formulation selon l'une quelconque des revendications 9 à 13, prévue pour un usage pédiatrique qui est reconstituée en une suspension aqueuse avant usage.

15. Formulation pédiatrique selon la revendication 14, qui comprend en outre des excipients extragranulaires choisis parmi un dessicant, des épaississants, des conservateurs, des édulcorants, des lubrifiants, des glissants et des arômes.

16. Formulation pédiatrique selon la revendication 15, dans laquelle l'épaississant est choisi parmi la gomme xanthane, la carboxyméthylcellulose sodique, le gel de silice ou l'hydroxypropylméthylcellulose.

17. Formulation pédiatrique selon la revendication 15 ou 16, dans laquelle l'arôme est un arôme fruité.

18. Formulation selon l'une quelconque des revendications 9 à 13, proposée sous forme de produit en sachet et contenant des granulés fluides en une dose unitaire adaptée, destinée à être reconstituée dans de l'eau pour constituer une formulation de sirop immédiatement avant usage.

19. Produit en sachet selon la revendication 18, qui comprend en outre des excipients choisis parmi des édulcorants, des épaississants, des conservateurs et des tampons.

20. Formulation selon l'une quelconque des revendications 9 à 13, proposée sous forme de comprimé, qui comprend en outre un délitant extragranulaire.

21. Formulation en comprimé selon la revendication 20, dans laquelle le délitant extragranulaire est l'hydroxypropylcellulose faiblement substituée.

22. Formulation en comprimé selon les revendications 20 ou 21, qui comprend en outre un lubrifiant extragranulaire.

23. Formulation en comprimé selon la revendication 22, dans laquelle le lubrifiant extragranulaire est le stéarate de magnésium.

24. Formulation en comprimé selon l'une quelconque des revendications 20 à 23, qui comprend des granulés d'amoxycilline qui consistent essentiellement en du trihydrate d'amoxycilline présent dans une proportion d'environ 97 % en poids et de N-vinyl-2-pyrrolidinone réticulée présente dans une proportion d'environ 3 % en poids ; des granulés d'amoxycilline et de clavulanate de potassium qui comprennent un rapport de 2:1 de trihydrate d'amoxycilline/ clavulanate de potassium où le rapport fait référence au poids du composé parent amoxycilline ou acide clavulanique ; du gel de silice présent dans une proportion d'environ 10 % en poids de clavulanate de potassium et d'environ 3 % en poids de N-vinyl-2-pyrrolidinone réticulée ; et des excipients extragranulaires comprenant : du gel de silice (1,0 à 1,2 % en poids du comprimé) ; de l'hydroxypropylcellulose faiblement substituée (5 à 6 % en poids du comprimé) ; de la silice colloïdale (0,1 à 0,3 % en poids du comprimé) ; et du stéarate de magnésium (0,3 à 0,4 % en poids du comprimé) ; ou de la N-vinyl-2-pyrrolidinone réticulée (5 à 8 % en poids du comprimé) ; et du stéarate de magnésium (0,3 à 0,4 % en poids du comprimé).

25. Formulation selon l'une quelconque des revendications 9 à 13, proposée sous forme d'un comprimé à croquer, qui comprend en outre un excipient extragranulaire, présent dans une proportion allant de 10 à 30 % sur la base du poids du comprimé final, utilisée pour former une base pouvant être croquée.

26. Formulation en comprimé selon la revendication 25, dans laquelle l'excipient extragranulaire est choisi parmi le mannitol, le sorbitol, le dextrose, le fructose ou le lactose, seuls ou combinés.

27. Formulation en comprimé selon les revendications 25 ou 26, qui comprend en outre un délitant présent dans une proportion allant de 5 à 30 % en se basant sur le poids du comprimé final.

28. Formulation en comprimé selon la revendication 27, dans laquelle le délitant est choisi parmi la cellulose microfine, la cellulose microcristalline, l'hydroxypropylcellulose, le glycolate d'amidon sodique ou la N-vinyl-2-pyrrolidinone réticulée.

29. Formulation en comprimé selon les revendications 25 ou 26, qui comprend en outre un couple effervescent, présent dans une proportion allant de 5 à 30 % sur la base du poids du comprimé final.

30. Formulation en comprimé selon la revendication 29, dans laquelle le couple effervescent comprend un acide solide choisi parmi l'acide citrique, le citrate monosodique, le citrate d'hydrogène sodique, l'acide tartrique, l'acide adipique, l'acide fumarique ou l'acide malique et un carbonate alcalin ou de métal alcalino-terreux ou un bicarbonate choisi parmi le (bi)carbonate de sodium, le (bi)carbonate de potassium, le (bi)carbonate de calcium ou le carbonate de glycérol sodique.

31. Formulation en comprimé selon l'une quelconque des revendications 20 à 30, qui comprend du trihydrate d'amoxycilline, du clavulanate de potassium et des excipients pharmaceutiquement acceptables dans laquelle le trihydrate d'amoxycilline est présent dans une quantité équivalente à 500 mg d'amoxycilline (acide libre) et le clavulanate de potassium est présent dans une quantité équivalente à 62,5 mg d'acide clavulanique.

32. Formulation en sachet pour une suspension selon la revendication 18, dans laquelle les poids et les proportions relatives des composants sont :

| | |
|---|---|
| Trihydrate d'amoxycilline 100 % théoriques (équivalant à 3000 mg d'amoxycilline totale) | 3443,22 mg |
| Clavulanate de potassium 100 % théoriques (équivalant à 375 mg d'acide clavulanique total) | 446,79 mg |
| N-vinyl-2-pyrrolidinone (CLPVP) (intragranulaire) | 103,29 mg |
| Gel de silice (intragranulaire) (Syloid AL) | 44,68 mg |
| Gel de silice (extragranulaire) (Syloid AL) | 500,00 mg |
| Gomme xanthane | 25,20 mg |
| Sel sodique de carboxyméthylcellulose | 250,00 mg |
| Benzoate de sodium | 51,00 mg |
| Silice colloïdale hydrophobe | 15,00 mg |
| Aspartame | 96,00 mg |
| Stéarate de magnésium | 10,00 mg |
| Arôme fraise | 150,00 mg |

dans laquelle les poids et les proportions relatives des composants peuvent varier autour des nombres répertoriés mais dans une plage de plus ou moins 10 %.

33. Procédé pour préparer une formulation selon l'une quelconque des revendications 9 à 32, qui comprend le mélange d'un premier ensemble de granulés selon les revendications 1 à 8 comprenant du trihydrate d'amoxycilline et du clavulanate de potassium conjointement avec un second ensemble de granulés comprenant du trihydrate d'amoxycilline et pas de clavulanate selon un rapport approprié avec d'autres excipients puis en outre, si nécessaire et si souhaité, le traitement du mélange pour obtenir la formulation finale souhaitée.